# EUROPEAN PATENT SPECIFICATION

(11) **EP 4 585 321 B1**
(45) Date of publication and mention of the grant of the patent: **07.10.2026**
(21) Application number: 25151839.5
(22) Date of filing: 14.01.2025
(51) Int. Cl.: B08B 9/30, B08B 9/34

(54) **SYSTEM FOR WASHING CONTAINERS FOR PHARMACEUTICAL USE AND METHOD FOR WASHING CONTAINERS FOR PHARMACEUTICAL USE**
SYSTEM ZUM WASCHEN VON BEHÄLTERN ZUR PHARMAZEUTISCHEN VERWENDUNG UND VERFAHREN ZUM WASCHEN VON BEHÄLTERN ZUR PHARMAZEUTISCHEN VERWENDUNG
SYSTÈME DE LAVAGE DE RÉCIPIENTS À USAGE PHARMACEUTIQUE ET PROCÉDÉ DE LAVAGE DE RÉCIPIENTS À USAGE PHARMACEUTIQUE

(30) Priority: 15.01.2024 IT 202400000582
(43) Date of publication of application: 16.07.2025
(73) Proprietor: NUOVA OMPI S.r.l. Unipersonale, 35017 Piombino Dese (PD) (IT); Gerresheimer Glas GmbH, 40468 Düsseldorf (DE)
(72) Inventor: TACCHINI, Paolo, I-40069 Zola Predosa (BO) (IT); ESCUDERO CUBILLO, Hector, I-40133 Bologna (BO) (IT)
(74) Representative: Porta & Consulenti Associati S.p.A.

(56) References cited:
- WO-A1-2020/234392
- US-A1- 2010 212 699

## Description

The present invention relates to a system for washing containers for pharmaceutical use and a method for washing containers for pharmaceutical use.

Containers for pharmaceutical use, such as syringes, cartridges, vials and the like, are subjected to washing operations with a heated fluid, usually water heated to a relatively high temperature, before being filled or packed.

These washing operations can be carried out in special washing plants with an entry station, one or more washing stations and an exit station. The containers to be washed are continuously fed to the entry station, washed at the washing station and taken to the entry station. In some cases, there may be pre-washing stations that precede the washing stations and in which the containers undergo pre-washing operations.

To increase the energy efficiency of washing operations and to reduce the amount of fluid used, the fluid that is used in the washing stations is recovered for use in the pre-washing stations.

In this regard, after the containers have been washed, the fluid is recovered and fed into a recovery tank. The recovery tank comprises a heater, usually an electric resistor, placed at the bottom of the tank to heat the recovered fluid before reusing it for pre-washing operations. From the recovery tank, the newly heated fluid is sent to the pre-washing stations from which, once used, it is drained from the plant.

When the washing plant has to be stopped, e.g. due to a malfunction of the container conveying device between the stations of the washing plant, or to perform a format change of the containers to be washed, or for any other reason, the supply of heated fluid is interrupted, the fluid is sent from the washing stations to the recovery tank and from the pre-washing stations to the drain. In this way, all the pipes necessary to transport the fluid are emptied and only in the recovery tank does fluid continue to be present. The recovery tank heater is kept active to keep the fluid contained therein at the right temperature and prevent it from cooling down.

When the washing system is restarted, the heated fluid supply is reactivated and the fluid contained in the recovery tank is sent to the pre-washing stations.

The Applicant noted that when the plant is restarted, especially after a prolonged stop, the pipes of the washing system cool down and need to be brought up to temperature again before the containers can be subjected to washing and pre-washing.

Indeed, the Applicant observed that when heated fluid passes through "cold" pipes, the fluid tends to cool down, losing its sterility and thus its suitability to effectively wash and pre-wash containers.

The Applicant verified that heating the pipes can be performed simply by running heated fluid through the pipes until the fluid releases sufficient heat to the pipes to bring them to a predetermined temperature.

The Applicant considered, however, that in this way it is necessary to reheat the fluid used to transfer heat to the pipes in order to bring it back to a temperature required to ensure its sterility, thus lengthening the time required for effectively restarting the washing plant.

The Applicant therefore felt the need to reduce the time required for effectively restarting the washing plant as much as possible, and realised that this need could be achieved by preventing at least part of the pipes of the washing system from cooling down excessively during the time the system was stopped.

The Applicant found that by preventing the cooling of at least part of the pipes of the washing system, it would be possible to reactivate the washing plant more quickly following a temporary stop.

Prior art document WO 2020/234392 A1 discloses a method for washing containers in accordance with the preamble of claim 1 and a system for washing containers in accordance with the preamble of claim 4.

The present invention thus relates, in a first aspect thereof, to a method for washing containers for pharmaceutical use comprising:
sending heated fluid to washing nozzles of at least one washing station through a washing circuit;
sending to a recovery tank fluid dispensed by said washing nozzles through said washing circuit;
sending fluid from said recovery tank to pre-washing nozzles of at least one pre-washing station through a pre-washing delivery branch of a pre-washing circuit;
wherein, in case of a temporary stop of the washing system, it is provided for:
   interrupting sending fluid to the pre-washing nozzles;
   placing a recirculation branch of the pre-washing circuit in fluid communication with said pre-washing delivery branch and with said recovery tank;
   recirculating fluid between the recovery tank, the pre-washing delivery branch and the recirculation branch, and heating said fluid while it recirculates.

The present invention relates, in a second aspect thereof, to a system for washing containers for pharmaceutical use comprising:
at least one washing station comprising a plurality of washing nozzles and a collection device for collecting fluid dispensed by said washing nozzles;
at least one pre-washing station comprising a plurality of pre-washing nozzles and a collection device for collecting fluid dispensed by said pre-washing nozzles;
a fluid recovery tank;
a washing circuit which places a fluid source in fluid communication with said washing nozzles and which places said fluid collection device of said at least one washing station in fluid communication with said recovery tank;
a pre-washing circuit comprising a pre-washing delivery branch placed in fluid communication with said recovery tank and selectively placed in fluid communication with said pre-washing nozzles and a pre-washing return branch placing said fluid collection device of said at least one pre-washing station in fluid communication with a drain;
a fluid heater placed on a portion of said pre-washing delivery branch and configured to heat fluid flowing in said portion of pre-washing delivery branch;
wherein said pre-washing circuit further comprises a recirculation branch placed in fluid communication with said recovery tank and selectively placed in fluid communication with said pre-washing delivery branch;
wherein when said recirculation branch is placed in fluid communication with said pre-washing delivery branch, said pre-washing delivery branch is not placed in fluid communication with said pre-washing nozzles.

In the solution proposed by the Applicant, in case of a temporary stop of the washing system, the pre-washing delivery branch is kept at a desired temperature and such that the fluid circulating therein is not cooled to a temperature below a fluid sterility temperature. In this way, the pre-washing operations can be reactivated almost immediately when the washing system is restarted.

Furthermore, as the pre-washing operations are carried out prior to the washing operations, when the washing system is restarted and during the pre-washing operations, the washing circuit can be restarted by, for example, running heated fluid through the washing circuit until the relevant pipes reach a temperature required to ensure the sterility of the washing fluid. As these operations can be carried out while (at restart) the containers are being pre-washed, they do not substantially delay the restart of the washing system.

The Applicant found that this not only allows the washing system to be restarted quickly in the event of a temporary stop, but also reduces the energy consumption of the washing system. As it is not necessary to recirculate hot fluid within the washing circuit during a temporary stop of the washing system, the use of energy (typically electricity) to heat any recirculation fluid for the washing circuit can be avoided. Only when the washing system is restarted, and while the containers are pre-washed, is heated fluid used to reactivate the washing circuit.

In the present description and the appended claims, the term "full use" when referring to the washing system means an operating condition of the washing system in which the containers are subjected to both washing and pre-washing operations.

In the present description and the appended claims, the term "temporary stop" when referring to the washing system means an operating condition of the washing system in which the containers are not subjected to washing or pre-washing operations. A temporary stop of the washing system is a temporary and short-term operating condition. A temporary stop of the washing system is a distinct operating condition from a complete stop. In the latter operating condition, the washing system is stopped for a much longer period of time.

In the present description and the appended claims, the term "restart" when referring to the washing system means an operating condition of the washing system that follows the temporary stop operating condition and precedes the full use operating condition.

Preferred features of the washing system and washing method are recited herein below, each of these features being able to be provided individually or in combination with the others.

Preferably, the fluid source is configured to deliver fluid heated to a predetermined temperature.

Preferably, the fluid supplied by the fluid source is water, more preferably it is distilled or demineralised water or osmosis water, and said predetermined temperature is comprised between 70 °C and 90 °C.

Preferably, in the event of a temporary stop of the washing system, the supply of fluid to the washing nozzles is interrupted.

Preferably, in the event of a temporary stop of the washing system, fluid contained in said fluid collection device of the washing station is drained into said recovery tank.

Preferably, said washing circuit comprises a washing delivery branch selectively placed in fluid communication with said fluid source and placed in fluid communication with said washing nozzles.

Preferably, in the event of a temporary stop of the washing system, the supply of fluid to said washing delivery branch is interrupted.

Preferably, when said recirculation branch is placed in fluid communication with said pre-washing delivery branch, said washing delivery branch is not placed in fluid communication with said fluid source.

Preferably, said washing circuit comprises a washing return branch which places in fluid communication said fluid-collecting device of said at least one washing station with said recovery tank.

Preferably, in the event of a temporary stop of the washing system, fluid is drained from said washing return branch to said recovery tank.

Thus, during the temporary stop of the washing system, the washing circuit is empty, i.e. there is no fluid in the washing circuit.

Preferably, a washing pump is mounted on the washing circuit, which increases the pressure of the fluid supplied by said fluid source.

The washing pump has the function of sending fluid to the washing nozzles.

Preferably, the washing pump is mounted on the washing delivery branch.

Preferably, a pre-washing pump is mounted on the pre-washing circuit to increase the pressure of the fluid contained in said recovery tank.

The pre-washing pump has the function of sending fluid from the recovery tank to the pre-washing nozzles.

Preferably, the pre-washing pump is mounted on the pre-washing delivery branch.

Preferably, heating said fluid while it recirculates comprises heating said fluid along said section of said pre-washing delivery branch.

The function of the fluid heater is to heat the fluid circulating in the pre-washing circuit during the pre-washing operations to a temperature required to ensure fluid sterility.

The fluid heater also has the function of heating the fluid that recirculates between the recovery tank, the pre-washing delivery branch and the recirculation branch, during temporary stop of the washing system, in order to keep this fluid at a temperature required to ensure sterility.

Preferably, said fluid heater is an electric immersion heater.

Preferably, said electric immersion heater is placed within said section of the pre-washing delivery branch.

In this way, the fluid circulating inside the pre-washing delivery branch is heated as it flows through the section of the pre-washing delivery branch in which the fluid heater is located.

Preferably, the pre-washing pump is placed between said recovery tank and said fluid heater.

Preferably, there is no fluid heater in the recovery tank.

The Applicant has verified that by heating the fluid flowing inside the pre-washing delivery branch and avoiding fluid heaters inside the recovery tank, it is possible to vary the temperature of the fluid more quickly, ensuring greater readiness of the washing system. In fact, the fluid heater acts on a reduced volume of fluid, precisely on a volume of fluid given by the volume of fluid flowing within the pre-washing delivery branch section. Otherwise, if the fluid heater were placed inside the recovery tank, the fluid heater would have to act on a much larger volume of fluid, namely on a volume of fluid given by the fluid contained in the recovery tank.

Preferably, the washing delivery branch comprises a washing main pipe fluidically connected to said fluid source and at least one washing dispensing pipe fluidically connected to said washing main pipe and said washing nozzles.

When the washing system is in full use, the fluid from the fluid source flows along the washing main pipe, reaches the washing supply pipe and then reaches the washing nozzles.

Preferably, an inlet valve is placed on said washing delivery branch and is switchable between an open position in which it places said source in fluid communication with said washing delivery branch and a closed position in which it interrupts fluid communication between said source and said washing delivery branch.

Preferably, when the washing system is in full use, the inlet valve is in the open position and fluid is dispensed from the washing nozzles onto the containers to be washed.

Preferably, in the event of a temporary stop of the washing system, the inlet valve is in the closed position and no fluid is dispensed from the washing nozzles onto the containers to be washed.

Preferably, the inlet valve is a solenoid valve.

Preferably, the washing pump is mounted on the washing main pipe.

Preferably, the inlet valve is interposed between the fluid source and the washing pump.

Preferably, a recirculation valve is provided between said recirculation branch and said pre-washing delivery branch, which is switchable between an open position in which it provides fluid communication between said pre-washing delivery branch and said recirculation branch and a closed position in which it interrupts fluid communication between said pre-washing delivery branch and said recirculation branch.

Preferably, when the washing system is in full use, the recirculation valve is in the closed position and no fluid is introduced into the recirculation branch.

Preferably, in the event of a temporary stop of the washing system, the recirculation valve is in the open position and fluid from the pre-washing delivery branch is fed into the recirculation branch and reaches the recovery tank.

Preferably, the recirculation valve is a solenoid valve.

Preferably, when said recirculation valve is in the open position said inlet valve is in the closed position and when said recirculation valve is in the closed position said inlet valve is in the open position.

Preferably, said pre-washing delivery branch comprises a pre-washing main pipe fluidically connected to said recovery tank and at least one pre-washing dispensing pipe fluidically connected to said pre-washing main pipe and said pre-washing nozzles.

Preferably, the section of the pre-washing delivery branch in which said fluid heater is placed is made from a portion of the main pre-washing pipe.

When the washing system is in full use, the fluid from the recovery tank flows along the pre-washing main pipe, reaches the pre-washing dispensing pipe and then reaches the pre-washing nozzles.

Preferably, a pre-washing dispensing valve is placed on said pre-washing dispensing pipe and is switchable between an open position in which it places said pre-washing main pipe and said pre-wash dispensing pipe in fluid communication and a closed position in which it interrupts fluid communication between said pre-washing main pipe and said pre-washing dispensing pipe.

Preferably, when the washing system is in full use, the pre-washing dispensing valve is in the open position and fluid is dispensed from the pre-washing nozzles onto the containers to be pre-washed.

Preferably, in the event of a temporary stop of the washing system, the pre-washing dispensing valve is in the closed position and no fluid is dispensed from the pre-washing nozzles onto the containers to be pre-washed.

Preferably, the pre-washing dispensing valve is a solenoid valve.

Preferably, when the pre-washing dispensing valve is in the open position, the recirculation valve is in the closed position.

Preferably, when the pre-washing dispensing valve is in the closed position, the recirculation valve is in the open position.

In this way, fluid from the recovery tank can reach the pre-washing nozzles and not reach the recirculation branch, or it can reach the recirculation branch and not reach the pre-washing nozzles.

Preferably, the pre-washing pump is mounted on the main pre-washing pipe.

Preferably, the pre-washing pump is mounted between the recovery tank and the pre-washing dispensing valve.

Preferably, said fluid heater is placed on said pre-washing main pipe of said pre-washing delivery branch.

Preferably, said fluid heater is mounted between the pre-washing pump and the pre-washing dispensing valve.

Preferably, said washing circuit and said pre-washing circuit are only in fluid communication with each other through said recovery tank.

Preferably, when the washing system is in full use, said washing circuit supplies with fluid said pre-washing circuit through said recovery tank.

Preferably, in case of a temporary stop of the washing system, said washing circuit does not supply the pre-washing circuit with fluid.

Preferably, said washing delivery branch comprises an auxiliary pipe fluidically connected with a drain and selectively fluidically connected with said washing main pipe.

Preferably, an auxiliary valve is provided between said auxiliary pipe and said washing main pipe, which is switchable between an open position in which it places said auxiliary pipe and said washing main pipe in fluid communication and a closed position in which it interrupts fluid communication between said washing main pipe and said auxiliary pipe.

Preferably, upon restarting the washing system after a temporary stop and before the washing system is in full use, the pre-washing delivery branch is placed in fluid communication with said pre-washing nozzles and said washing main pipe is placed in fluid communication with said auxiliary pipe.

In this way, during the restart of the washing system, the containers are pre-washed while at least part of said washing delivery branch is heated to a temperature required to maintain sterility of the fluid.

When the washing main pipe is heated to the correct temperature, the auxiliary valve is placed in the closed position allowing the activation of the washing nozzles and full use of the washing system.

Further, by keeping the pre-washing circuit at least partially filled with water, pressure surges, commonly known as "water hammer", which would lead to mechanical damage of the circuit piping, are greatly reduced when restarting.

Further characteristics and advantages of the present invention will become clearer from the following detailed description of preferred embodiments thereof, with reference to the accompanying drawings and given for indicative and non-limiting purposes. In such drawings:
- Figure 1 schematically shows a washing system according to the present invention and in a first operating condition;
- Figures 2 to 6 schematically show the washing system in Figure 1 under further operating conditions;
- Figure 7 represents a detail of the washing system in Figure 1; and
- Figure 8 represents a connection diagram between some components of the washing system in Figure 1.

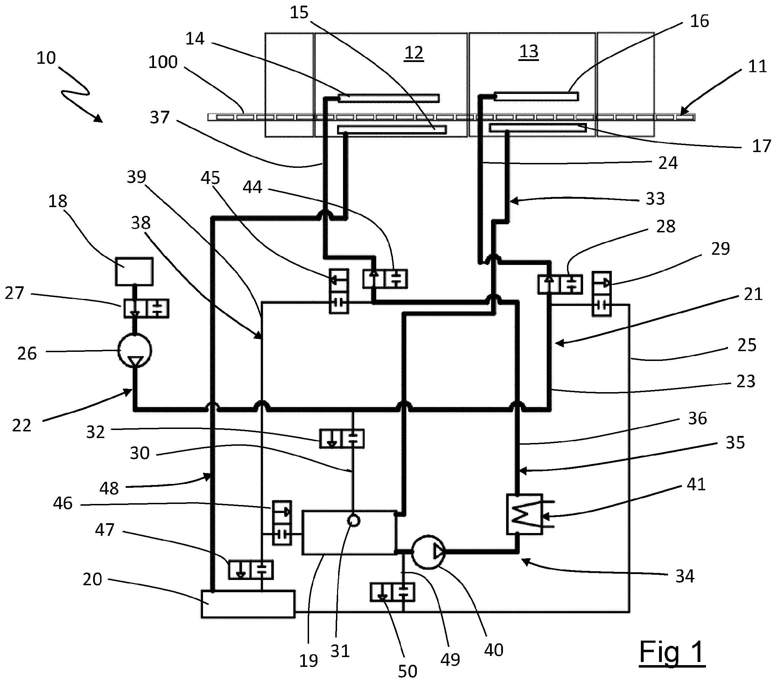

A washing system according to the present invention is shown in Figure 1 by number 10.

The system 10 comprises a conveyor 11 configured to transport containers for pharmaceutical use 100 along a conveyor line.

The system 10 further comprises a pre-washing station 12 and a washing station 13. The conveyor 11 passes through pre-washing station 12 and the washing station 13 in such a way that containers for pharmaceutical use 100 pass through the pre-washing station 12 and the washing station 13 in succession, as shown in Figures 1 to 6.

The pre-washing station 12 comprises a plurality of pre-washing nozzles 14 configured to dispense fluid onto the containers 100. The pre-washing nozzles 14 are positioned in such a way that they can dispense fluid onto all the surfaces of the containers 100 that are to be pre-washed. By way of example, the pre-washing nozzles 14 may be arranged in arrays along the path followed by the conveyor 11 and dispense fluid along a plurality of different dispensing directions.

In the pre-washing station 12 there is a collection device 15 configured to collect the fluid dispensed by the pre-washing nozzles 14 during the pre-washing operations of the containers 100. The collection device 15 can, for example, be implemented by one or more collection tanks placed inside the pre-washing station 12.

The pre-washing station 12 has the function of performing a preventive wash of the containers 100.

The washing station 13 is placed after the pre-washing station 12 along the conveying line followed by the conveyor 11. The containers 100 leaving the pre-washing station 12 enter the washing station 13.

The washing station 13 comprises a plurality of washing nozzles 16 configured to dispense fluid onto the containers 100. The washing nozzles 16 are positioned in such a way that they can dispense fluid onto all the surfaces of the containers 100 that need to be washed. By way of example, the washing nozzles 16 may be arranged in arrays along the path followed by the conveyor 11 and dispense fluid along a plurality of different dispensing directions.

In the washing station 13 there is a collection device 17 configured to collect the fluid dispensed by the washing nozzles 16 during the washing operations of the containers 100. The collection device 17 can, for example, be implemented by one or more collection tanks placed inside the washing station 13.

The pre-washing station 12 and the washing station 13 can be structurally identical.

The system 10 further comprises a source 18 of fluid heated to a predetermined temperature, a recovery tank 19 and one or more fluid drains 20.

To supply fluid to the washing nozzles 16, the system 10 comprises a washing circuit 21 that selectively places the source 18 in fluid communication with the washing nozzles 16. The washing circuit 21 also selectively places the collection device 17 of the washing station 13 in fluid communication with the recovery tank 19.

The washing circuit 21 comprises a washing delivery branch 22 which extends between the source 18 and the washing nozzles 16. The washing delivery branch 22 comprises a washing main pipe 23 connected to the source 18, and a washing dispensing pipe 24 connected to the washing main pipe 23 and the washing nozzles 16. The washing delivery branch 22 also comprises an auxiliary pipe 25 placed in fluid connection with one of said drains 20 and selectively placed in fluid connection with the washing main pipe 23.

A washing pump 26 regulated in flow rate or pressure is placed on the washing main pipe 23 after the source 18, which pushes fluid into the washing delivery branch 22 to ensure correct fluid flow rate and delivery pressure from the washing nozzles 16. An inlet valve 27 is also provided between the washing pump 26 and the source 18 which can be switched between an open condition in which it allows fluid to flow from the source 18 to the washing pump 26 and a closed condition in which it interrupts fluid flow from the source 18 to the washing pump 26. The inlet valve 27 is a solenoid valve.

On the washing dispensing pipe 24, between the washing main pipe 23 and the washing nozzles 16, there is a washing dispensing valve 28 which is switchable between an open condition in which it allows fluid to flow from the washing main pipe 23 to the washing nozzles 16 and a closed condition in which it interrupts fluid flow from the washing main pipe 23 to the washing nozzles 16. The washing dispensing valve 28 is placed in the immediate vicinity of the washing main pipe 23. The washing dispensing valve 28 is a solenoid valve.

On the auxiliary pipe 25, between the washing main pipe 23 and the drain 20, there is an auxiliary valve 29 which can be switched between an open condition in which it allows fluid to flow from the washing main pipe 23 to the drain 20 and a closed condition in which it interrupts fluid flow from the washing main pipe 23 to the drain 20. The auxiliary valve 29 is placed in the immediate vicinity of the washing main pipe 23. The auxiliary valve 29 is a solenoid valve.

The washing delivery branch 22 also comprises a tank filling pipe 30 that selectively places the source 18 in fluid communication with the recovery tank 19. The tank filling pipe 30 is connected to the washing main pipe 23 between the washing pump 26 and the washing nozzles 16. The tank filling pipe 30 is interposed between the washing pump 26, the washing dispensing valve 28 and the auxiliary valve 29. Inside the recovery tank 19 there is a sprayball 31 hydraulically connected to the tank filling pipe 30.

On the tank filling pipe 30 between the washing main line 23 and the sprayball 31, there is a tank valve 32 which is switchable between an open condition in which it allows fluid to flow from the washing main line 23 to the sprayball 31 and a closed condition in which it interrupts fluid flow from the washing main line 23 to the sprayball 31. The tank valve 32 is placed in the immediate vicinity of the washing main pipe 23. The tank valve 32 is a solenoid valve.

The washing circuit 21 also comprises a washing return branch 33 that places the collection device 17 of the washing station 13 in fluid communication with the recovery tank 19. A return pump (not shown) can be provided on the washing return branch 33.

The pre-washing nozzles 14 are supplied with fluid which has been introduced from the collection device 17 of the washing station 13 into the recovery tank 19.

For this purpose, the system 10 comprises a pre-washing circuit 34. The pre-washing circuit 34 selectively places the recovery tank 19 in fluid communication with the pre-washing nozzles 14. The pre-washing circuit 34 also selectively places the collection device 15 of the pre-washing station 12 in fluid communication with one of the drains 20.

The pre-washing circuit 34 comprises a pre-washing delivery branch 35 which runs between the recovery tank 19 and the pre-washing nozzles 14. The pre-washing delivery branch 35 comprises a pre-washing main pipe 36 connected to the recovery tank 19, and a pre-washing dispensing pipe 37 connected to the pre-washing main pipe 36 and the pre-washing nozzles 14.

The pre-washing circuit further comprises a recirculation branch 38 selectively placed in fluid connection with the pre-washing delivery branch 35 and selectively placed in fluid connection with one of the drains 20 or the recovery tank 19. The recirculation branch 38 comprises a recirculation pipe 39 extending from the main pre-washing pipe 36 to the recovery tank 19 and to one of the drains 20.

A pre-washing pump 40 regulated in flow rate or pressure is placed on the pre-washing main pipe 36, after the recovery tank 19, which draws fluid from the recovery tank 19 and pushes it into the pre-washing delivery branch 35 to ensure a correct fluid flow rate and delivery pressure from the pre-washing nozzles 14. The pre-washing pump 40 is located near the recirculation tank 19.

A fluid heater 41 acts on the pre-washing main pipe 36 after the pre-washing pump 40 to heat the fluid being drawn from the recovery tank 19.

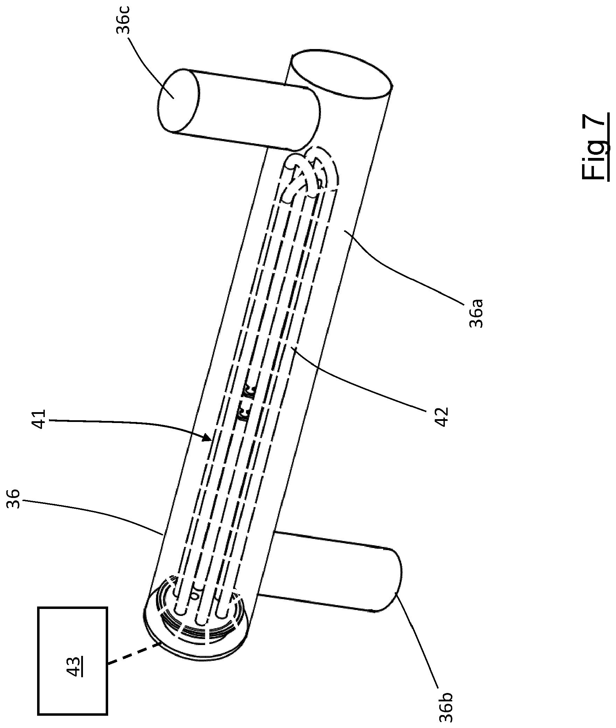

As best illustrated in Figure 7, the fluid heater 41 is placed within a portion 36a of the main pre-washing pipe 36. This portion 36a comprises a fluid inlet 36b and a fluid outlet 36c. The fluid heater 41 comprises an immersion heating element 42 placed between the fluid inlet 36b and the fluid outlet 36c. The fluid entering the pre-washing main pipe portion 36a through the fluid inlet 36a laps the heating element 42 which yields heat to the fluid thus heating it. The heated fluid then exits from the fluid outlet 36c of the portion 36a. The heating element 42 is, in the preferred embodiment of the invention, an electric resistor having an extension within the portion 36a preferably of about 50 centimetres. Preferably, the fluid heater 41 absorbs a maximum power of 5 kW. The fluid heater 41 is controlled by a power controller 43 (depicted in Figure 7) for electrical resistive loads.

On the pre-washing dispensing pipe 37, between the pre-washing main pipe 36 and the pre-washing nozzles 14, there is a pre-washing dispensing valve 44 switchable between an open condition in which it allows fluid to flow from the pre-washing main pipe 36 to the pre-washing nozzles 14 and a closed condition in which it interrupts fluid flow from the pre-washing main pipe 36 to the pre-washing nozzles 14. The pre-washing dispensing valve 44 is placed in the immediate vicinity to the pre-washing main pipe 36. The pre-washing dispensing valve 44 is a solenoid valve.

On the recirculation pipe 39, between the pre-washing main pipe 36 and the recovery tank 19 and the drains 20, there is a recirculation valve 45 which is switchable between an open condition in which it allows fluid to flow from the pre-washing main pipe 36 to the recovery tank 19 and drains 20 and a closed condition in which it interrupts fluid flow from the pre-washing main pipe 36 to the recovery tank 19 and the drains 20. The recirculation valve 45 is placed in the immediate vicinity to the pre-washing main pipe 36. The recirculation valve 45 is a solenoid valve.

On the recirculation pipe 39, between the recirculation valve 45 and the recovery tank 19, there is a recovery valve 46 which can be switched between an open condition in which it allows fluid to flow to the recovery tank 19 and a closed condition in which it interrupts fluid flow to the recovery tank 19. The recovery valve 46 is placed in the immediate vicinity of the recovery tank 19. The recovery valve 46 is a solenoid valve.

On the recirculation pipe 39, between the recirculation valve 45 and a drain 20, there is a first drain valve 47 which can be switched between an open condition in which it allows fluid to flow to the drain 20 and a closed condition in which it interrupts fluid flow to the drain 20. The first drain valve 47 is located in the immediate vicinity of the drain 20. The first drain valve 47 is a solenoid valve.

The pre-washing circuit 34 further comprises a pre-washing return branch 48 which connects the collection device 15 of the pre-washing station 12 with the drain 20. A return pump (not shown) can be provided on the pre-washing return branch 48.

The recovery tank 19 is also selectively hydraulically connected to the drain 20. In this regard, an outlet pipe 49 connects the recovery tank 19 with the drain 20. On the drain pipe 49 there is a second drain valve 50 which can be switched between an open condition in which it allows fluid to flow from the recovery tank 19 to the drain 20 and a closed condition in which it interrupts fluid flow from the recovery tank 19 to the drain 20. The second drain valve 50 is a solenoid valve.

The operation of the system 10 is managed and controlled by a command and control unit 51, e.g. a microprocessor.

The activation of the pumps, solenoid valves and fluid heater 41 is controlled by the command and control unit 51.

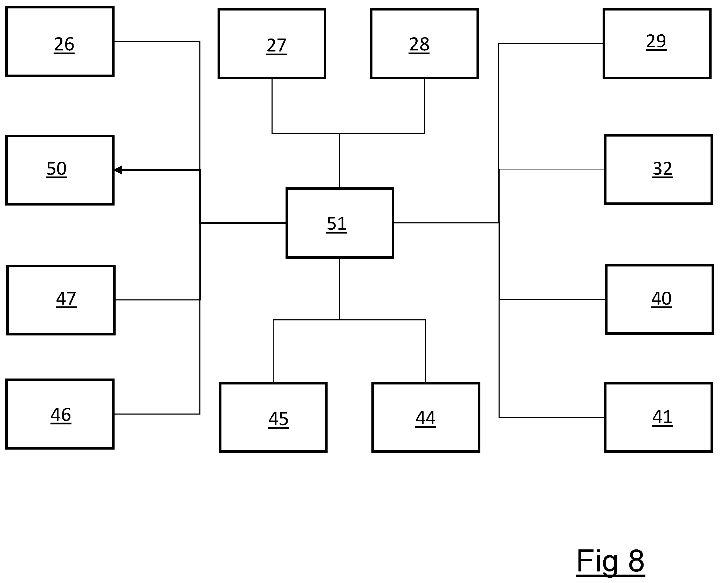

As schematically illustrated in Figure 8, the command and control unit 51 is placed in electrical signal and actuation connection with the washing pump. The command and control unit 51 is also placed in electrical signal and actuation connection with the inlet valve 27. The command and control unit 51 is also placed in electrical signal and actuation connection with the washing dispensing valve 28. The command and control unit 51 is also placed in electrical signal and actuation connection with auxiliary valve 29. The command and control unit 51 is also placed in electrical signal and actuation connection with the tank valve 32. The command and control unit 51 is also placed in electrical signal and actuation connection with the pre-washing pump 40. The command and control unit 51 is also placed in electrical signal and actuation connection with the fluid heater 41. The command and control unit 51 is also placed in electrical signal and actuation connection with the pre-washing dispensing valve 44. The command and control unit 51 is also placed in electrical signal and actuation connection with the recirculation valve 45. The command and control unit 51 is also placed in electrical signal and actuation connection with the recovery valve 46. The command and control unit 51 is also placed in electrical signal and actuation connection with the first drain valve 47. The command and control unit 51 is also placed in electrical signal and actuation connection with the second drain valve 50.

A method for washing containers for pharmaceutical use in accordance with the present invention will hereinafter be described by explicit reference to the above-mentioned washing system 10. Clearly, this method can be implemented by washing systems that are not necessarily identical to the one described above.

Figure 1 shows full use of the washing system, i.e. a steady state use condition during which the containers 100 are picked up and washed. In Figure 1, the pipes within which fluid flows have been shown with a thicker line.

In this condition, fluid heated to a sterilisation temperature from the source 18 is introduced into the washing delivery branch 22 and reaches the washing nozzles 16 which dispense fluid onto the containers 100 (already pre-washed as described in more detail below). For this purpose, the inlet valve 27 is in the open position and the washing pump 26 is activated. The tank valve 32 is in the closed position. The auxiliary valve 29 is in the closed position. The washing dispensing valve 28 is in the open position. In this condition, the heated fluid from the source 18 flows through the washing main pipe 23, reaches and flows through the washing dispensing pipe 28 and exits through the washing nozzles 16.

In the full use condition, the fluid dispensed by the washing nozzles 16 collects in the collection device 17 of the washing station 13 and reaches the recovery tank 19 via the washing return branch 33.

From the recovery tank 18, fluid is introduced into the pre-washing circuit 34 to reach the pre-washing nozzles 14 and be dispensed by them. For this purpose, the pre-washing pump 40 is switched on and the fluid heater 41 is energised. The pre-washing dispensing valve 37 is in the open position. The recirculation valve 45 is in the closed position. The recovery valve 46 and the first drain valve 47 may be in the open or closed position (preferably in the closed position) since no fluid reaches them from the recirculation branch 38. In this condition, fluid from the recovery tank 19 travels through the pre-washing main pipe 36, is heated in the portion 36a of the pre-washing main pipe 36 by the fluid heater 41, reaches and travels through the pre-washing dispensing pipe 37 and exits the pre-washing nozzles 14.

In the full use condition, the fluid dispensed by the pre-washing nozzles 14 collects in the collection device 15 of the pre-washing station 12 and reaches the drain via the pre-washing return branch 48.

Therefore, the heated fluid used for washing the containers 100 is reused, being heated again, for the pre-washing of the containers 100. The temperature of the fluid exiting the source 18 is such that sterility of the fluid is guaranteed. Likewise, the temperature at which the fluid heater 41 heats the fluid from the recovery tank 19 is such that the sterility of the fluid is guaranteed. Preferably, the temperature of the fluid exiting the source 18 is equal to the temperature at which the fluid is heated by the fluid heater 41. When water (distilled, demineralised or osmoticised) is used, this temperature is around 80 °C.

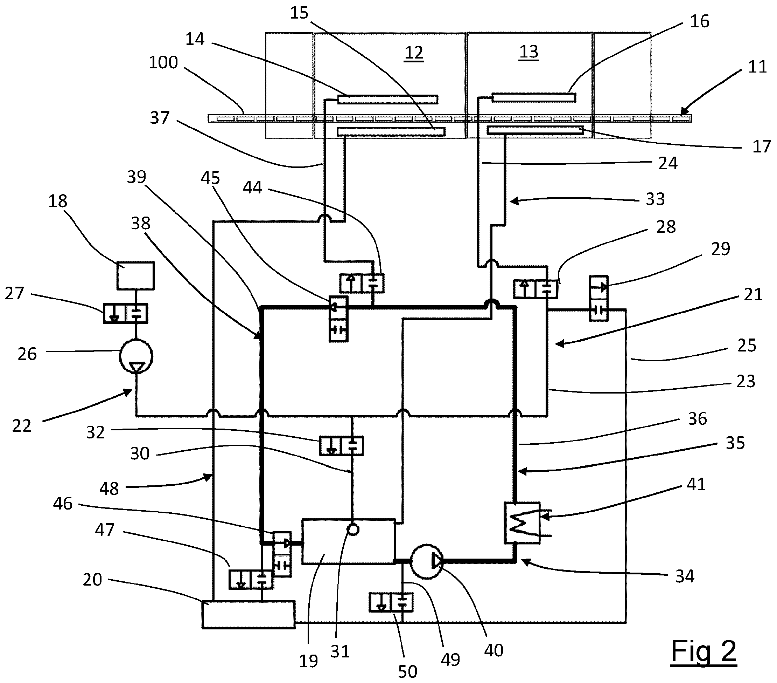

Figure 2 shows a temporary stop condition of the washing system 10, i.e. a condition of temporary suspension of the washing and pre-washing operations of the containers 100.

In this condition, the supply of fluid from the pre-washing nozzle 14 and the washing nozzles 16 is interrupted and fluid is recirculated, being heated to a temperature required to ensure sterility, between the recovery tank 19, the pre-washing delivery branch 35 and the recirculation branch 38. The temperature to which the recirculated fluid is heated is preferably equal to the temperature to which the fluid is heated during full use of the washing system.

For this purpose, the pre-washing pump 40 is switched on and the fluid heater 41 is energised. The pre-washing dispensing valve 37 is in the closed position. The recirculation valve 45 is in the open position. The recovery valve 46 is in the open position. The first drain valve 47 is in the closed position. In this condition, fluid from the recovery tank 19 travels through the pre-washing main pipe 36, is heated in the portion 36a of the pre-washing main pipe 36 by the fluid heater 41, reaches and travels through the recirculation pipe 39 and returns to the recovery tank 19.

In this condition, the inlet valve 27 is in the closed position and the washing pump 26 is deactivated. No fluid is therefore flowing inside the washing circuit 21. The fluid present in the washing circuit 21 at the time the inlet valve 27 closes and the washing pump 26 is switched off (i.e. when the washing system stops) collects in the collection device 17 of the washing station 13 and is drained into the recovery tank 19 via the washing return branch 33.

This condition is maintained until the washing system is restarted. Upon restarting, the fluid flowing within the pre-washing delivery branch 35, as well as the pipes of the latter, are at the temperature required to ensure the sterility of the fluid. In fact, it should be noted that in the accompanying figures the pre-washing dispensing pipe 37 has been depicted with a slightly smaller extension than the extension of the main pre-washing pipe 36 for the sake of representation. In the preferred embodiment of the invention, the extension of the pre-washing dispensing pipe 37 is much smaller than the extension of the pre-washing main pipe 36. Preferably, an overall volume of the pre-washing dispensing pipe 37 is at least 3 times lower than an overall volume of the pre-washing main pipe 36, more preferably it is at least 5 times lower than an overall volume of the pre-washing main pipe 36, more preferably it is at least 8 times lower than an overall volume of the pre-washing main pipe 36, more preferably it is at least 12 times lower than an overall volume of the pre-washing main pipe 36.

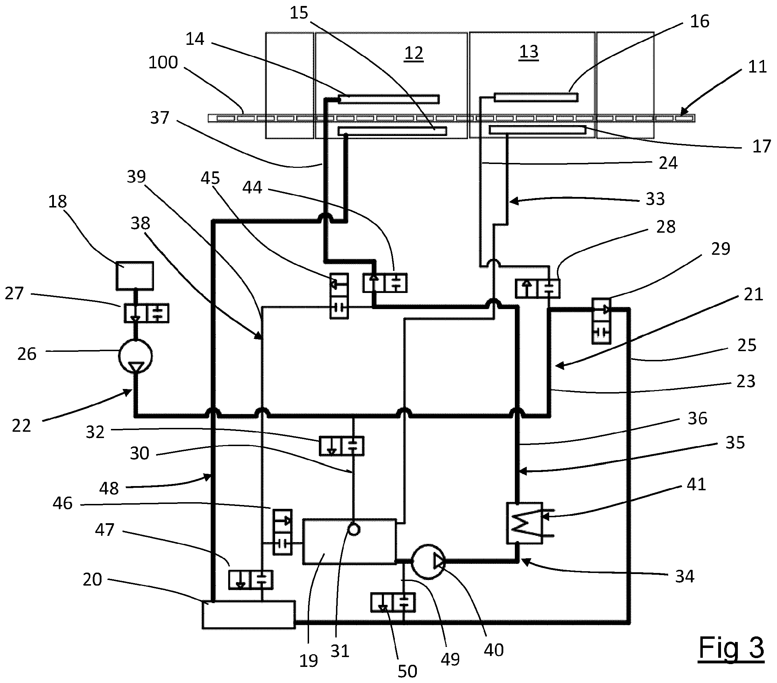

Upon restarting, illustrated in Figure 3 where the pipes within which fluid flows have been shown with a thicker line, the pre-washing circuit 34 is returned to the same condition as the full use condition (described above).

Upon restarting, the washing delivery branch 22 is at a lower temperature than the temperature required to ensure sterility of the fluid. Therefore, the washing delivery branch 22 is heated to a temperature required to ensure sterility of the fluid before fluid is dispensed from the washing nozzles 16. This action is performed by flowing heated fluid from the source 18 into the washing main pipe 23 and draining this fluid into the drain 20 until the washing main pipe 23 reaches a temperature required to ensure sterility of the fluid. While this action takes place, the containers 100 are subjected to pre-washing. Again, in the accompanying figures the washing dispensing pipe 24 has been depicted with a slightly smaller extension than the extension of the washing main pipe 23 for the sake of representation. In the preferred embodiment of the invention, the extension of the washing dispensing pipe 24 is much smaller than the extension of the washing main pipe 23. Preferably, an overall volume of the washing dispensing pipe 24 is at least 3 times lower than an overall volume of the washing main pipe 23, more preferably it is at least 5 times lower than an overall volume of the washing main pipe 23, more preferably it is at least 8 times lower than an overall volume of the pre-washing main pipe 23, more preferably it is at least 12 times lower than an overall volume of the washing main pipe 23.

During restart, the washing delivery branch 22 is heated as follows. The inlet valve 27 is in the open position and the washing pump 26 is active. The tank valve 32 is in the closed position. The auxiliary valve 29 is placed in the open position. The washing dispensing valve 28 is in the closed position. In this condition, the heated fluid from the source 18 flows through the washing main pipe 23, reaches and flows through the auxiliary pipe 25 and is drained into the drain 20.

When the washing delivery branch 22 is heated to the temperature required to ensure sterility of the fluid, the washing circuit 21 is also returned to the same condition as the full use condition (described above).

After a complete stop of the washing system 10 in which no fluid flows in the washing system, or when the washing system is started up for the first time, the washing system piping must be heated before the washing system is put into full use.

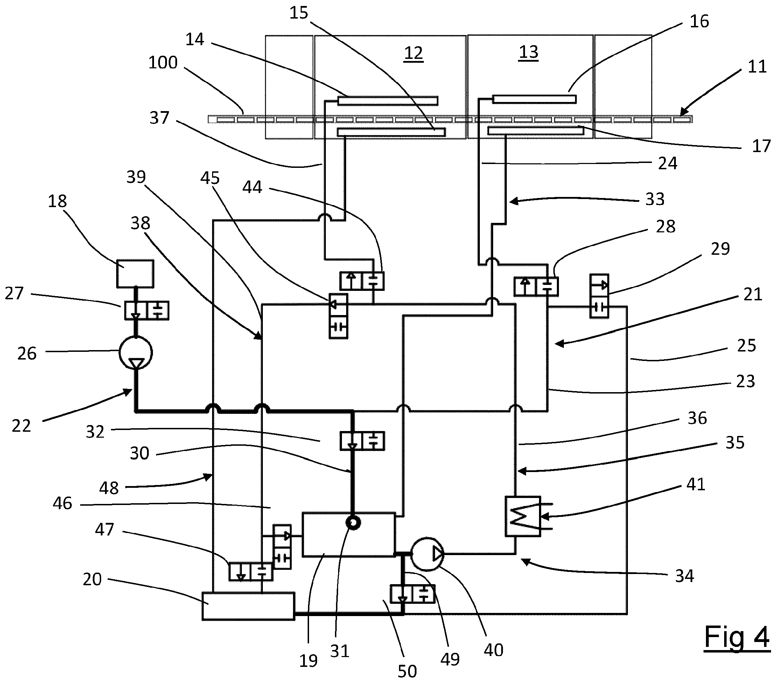

Figures 4 to 6 illustrate a sequence of operations that are carried out to heat the pipes of the washing system to a temperature required to ensure the sterility of the fluid.

As shown in Figure 4, where the pipes within which fluid flows have been depicted with a thicker line, heated fluid from the source 18 is first sent into the recovery tank 19 to heat the latter. For this purpose, the inlet valve 27 is placed in the open position and the washing pump 26 is activated. The tank valve 32 is in the open position. The auxiliary valve 29 is placed in the closed position. The washing dispensing valve 28 is in the closed position. The pre-washing pump 40 is deactivated. The second drain valve 50 is in the open position. In this condition, the heated fluid from the source 18 travels through part of the washing main pipe 23, travels through the tank filling pipe 30, reaches the sprayball 31 which dispenses heated fluid into the recovery tank 19. Fluid entering the recovery tank 19 is drained into the drain 20.

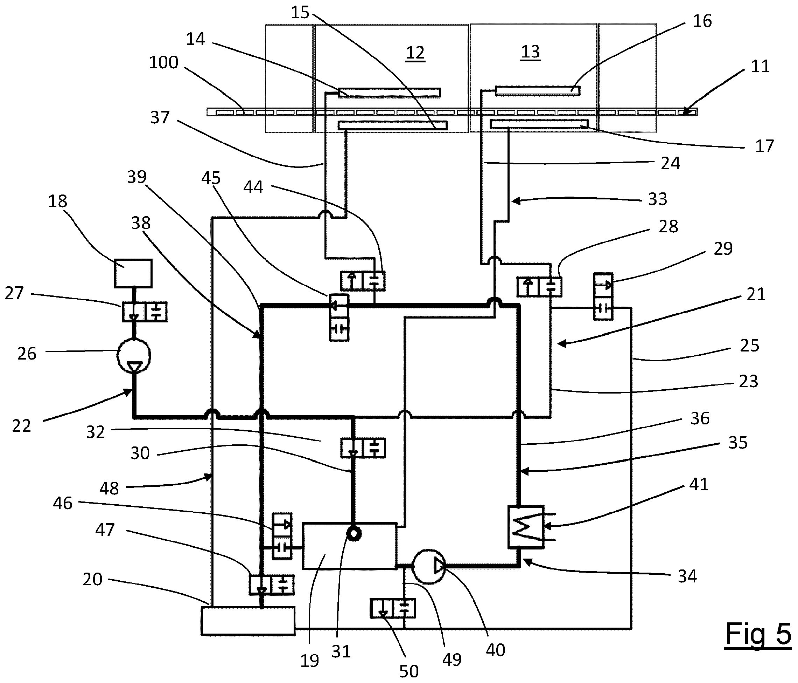

When the recovery tank 19 reaches the temperature required to ensure sterility, the recovery tank 19 is supplied with enough fluid from the source 19 to maintain a constant level in the recovery tank 19. The heated fluid sent from the source 18 to the recovery reservoir 19 is sent into the pre-washing delivery branch 35 until the pre-washing delivery branch 35 reaches a temperature required to ensure sterility of the fluid. This condition is illustrated in Figure 5. In this condition, the inlet valve 27 is in the open position and the washing pump 26 is active. The tank valve 32 is in the open position. The auxiliary valve 29 is placed in the closed position. The washing dispensing valve 28 is in the closed position. The pre-washing pump 40 is activated. The second drain valve 50 is in the closed position. The fluid heater 41 is energised. The pre-washing dispensing valve 37 is in the closed position. The recirculation valve 45 is in the open position. The recovery valve 46 is in the closed position. The first drain valve 47 is in the open position. In this condition, the fluid introduced into the recovery tank 19 and coming directly from the source 18 travels through the pre-washing main pipe 36, is heated in the portion 36a of the pre-washing main pipe 36 by the fluid heater 41, reaches and travels through the recirculation pipe 39 and is drained into the drain 20.

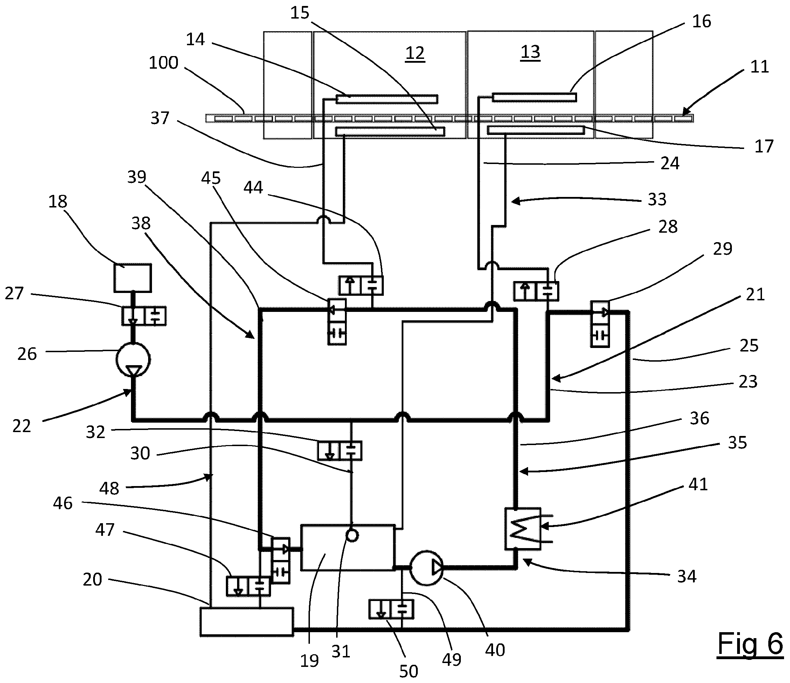

When the pre-washing delivery branch 35 reaches a temperature required to ensure sterility of the fluid, heated fluid is sent from the source 18 into the washing delivery branch 22, keeping the recovery tank 19 and the pre-washing delivery branch 35 at the temperature reached, as shown in Figure 6. In this condition, the inlet valve 27 is in the open position and the washing pump 26 is active. The tank valve 32 is in the closed position. The auxiliary valve 29 is placed in the open position. The washing dispensing valve 28 is in the closed position. The pre-washing pump 40 is activated. The second drain valve 50 is in the closed position. The fluid heater 41 is energised. The pre-washing dispensing valve 37 is in the closed position. The recirculation valve 45 is in the open position. The recovery valve 46 is in the open position. The first drain valve 47 is in the closed position. In this condition, the pre-washing circuit 34 is brought into the same use condition as the temporary stop condition (described above) and the washing circuit 21 is brought into the same use condition as the restart condition (described above).

When the pipes of the washing system reach the temperature required to ensure the sterility of the fluid, the washing system is brought into a full use condition.

## Claims

1. Method for washing containers (100) for pharmaceutical use comprising:
sending heated fluid to washing nozzles (16) of at least one washing station (13) through a washing circuit (21);
sending to a recovery tank (19) fluid dispensed from said washing nozzles (16) through said washing circuit (21);
sending fluid from said recovery tank (19) to pre-washing nozzles (14) of at least one pre-washing station (12) through a pre-washing delivery branch (35) of a pre-washing circuit (34);
wherein, in case of a temporary stop of the washing system, it is provided for:
interrupting sending fluid to pre-washing nozzles (14);
**characterised by**
placing a recirculation branch (38) of the pre-washing circuit (34) in fluid communication with said pre-washing delivery branch (35) and with said recovery tank (19);
recirculating fluid between the recovery tank (19), the pre-washing delivery branch (35) and the recirculation branch (38), and heating said fluid while it recirculates.

2. Method according to claim 1, wherein in case of a temporary stop of the washing system, it is provided for stopping sending fluid to the washing nozzles (16).

3. Method according to claim 1 or 2, wherein heating said fluid while it recirculates comprises heating said fluid along a portion of said pre-washing delivery branch (35).

4. System (10) for washing containers for pharmaceutical use comprising:
at least one washing station (13) comprising a plurality of washing nozzles (16) and a collection device (17) for collecting fluid dispensed from said washing nozzles (16);
at least one pre-washing station (12) comprising a plurality of pre-washing nozzles (14) and one collection device (15) for collecting fluid dispensed from said pre-washing nozzles (14);
a fluid recovery tank (19);
a washing circuit (21) which places a fluid source (18) in fluid communication with said washing nozzles (16) and which places said fluid collection device (17) of said at least one washing station (13) in fluid communication with said recovery tank (19);
a pre-washing circuit (34) comprising a pre-washing delivery branch (35) placed in fluid communication with said recovery tank (19) and selectively placed in fluid communication with said pre-washing nozzles (14) and a pre-washing return branch (48) placing said fluid collection device (15) of said at least one pre-washing station (12) in fluid communication with a drain (20);
a fluid heater (41) placed on a portion of said pre-washing delivery branch (35) and configured to heat fluid flowing in said portion of pre-washing delivery branch (35);
**characterised in that** said pre-washing circuit (34) further comprises a recirculation branch (38) placed in fluid communication with said recovery tank (19) and selectively placed in fluid communication with said pre-washing delivery branch (35);
and **in that** when said recirculation branch (38) is placed in fluid communication with said pre-washing delivery branch (35), said pre-washing delivery branch (35) is not placed in fluid communication with said pre-washing nozzles (14).

5. Washing system (10) according to claim 4, wherein said washing circuit (21) comprises a washing delivery branch (22) selectively placed in fluid communication with said fluid source (18) and placed in fluid communication with said washing nozzles (16) and a washing return branch (33) which places said fluid collection device (17) of said at least one washing station (13) in fluid communication with said recovery tank (19).

6. Washing system (10) according to claim 5, wherein when said recirculation branch (38) is placed in fluid communication with said pre-washing delivery branch (35), said washing delivery branch (22) is not placed in fluid communication with said fluid source (18)

7. Washing system (10) according to any one of claims 4 to 6 wherein said fluid source (18) is configured to dispense fluid heated to a predetermined temperature.

8. Washing system (10) according to any one of claims 4 to 7, wherein between said recirculation branch (38) and said pre-washing delivery branch (35) it is provided a recirculation valve (45) switchable between an open position in which it places said pre-washing delivery branch (35) in fluid communication with said recirculation branch (38) and a closed position in which it interrupts the fluid communication between said pre-washing delivery branch (35) and said recirculation branch (38).

9. Washing system (10) according to any one of claims 4 to 8 wherein said pre-washing delivery branch (35) comprises a pre-washing main pipe (36) fluidically connected with said recovery tank (19) and at least one pre-washing dispensing pipe (37) fluidically connected with said pre-washing main pipe (36) and with said pre-washing nozzles (14); a pre-washing dispensing valve (44) being placed on said pre-washing dispensing pipe (37) and being switchable between an open position in which it places said main pre-washing pipe (36) in fluid communication with said pre-washing dispensing pipe (37) and a closed position in which it interrupts the fluid communication between said main pre-washing pipe (36) and said pre-washing dispensing pipe (37).

10. Washing system (10) according to claim 8 and 9, wherein when said recirculation valve (45) is in the open position said pre-washing dispensing valve (44) is in the closed position and when said recirculation valve (45) is in the closed position said pre-washing dispensing valve (44) is in the open position.

11. Washing system (10) according to claim 4 and 9, wherein said fluid heater (41) is placed on said pre-washing main pipe (36) of said pre-washing delivery branch (35).

12. Washing system (10) according to any one of claims 4 to 11, wherein said washing circuit (21) and said pre-washing circuit (34) are in fluid communication with each other only through said recovery tank (19).

## Patentansprüche

1. Verfahren zum Waschen von Behältern (100) für pharmazeutische Zwecke, umfassend:
das Zuführen von erhitzter Flüssigkeit zu Waschdüsen (16) mindestens einer Waschstation (13) über einen Waschkreislauf (21);
das Zuführen der aus den Waschdüsen (16) über den Waschkreislauf (21) abgegebenen Flüssigkeit in einen Rückgewinnungstank (19);
das Zuführen von Flüssigkeit aus dem Rückgewinnungstank (19) über einen Vorwasch-Zuführzweig (35) eines Vorwaschkreislaufs (34) zu Vorwaschdüsen (14) mindestens einer Vorwaschstation (12);
wobei im Falle eines vorübergehenden Stillstands des Waschsystems Folgendes vorgesehen ist:
das Unterbrechen des Zuführens von Flüssigkeit zu den Vorwaschdüsen (14); **gekennzeichnet durch**
das In-Fluidverbindung-Bringen eines Rückführzweigs (38) des Vorwaschkreislaufs (34) mit dem Vorwasch-Zuführzweig (35) und mit dem Rückgewinnungstank (19);
das Rückführen von Flüssigkeit zwischen dem Rückgewinnungstank (19), dem Vorwasch-Zuführzweig (35) und dem Rückführzweig (38) sowie Erhitzen der Flüssigkeit während ihrer Rückführung.

2. Verfahren nach Anspruch 1, wobei im Falle eines vorübergehenden Stillstands des Waschsystems vorgesehen ist, das Zuführen von Flüssigkeit zu den Waschdüsen (16) zu unterbrechen.

3. Verfahren nach Anspruch 1 oder 2, wobei das Erhitzen der Flüssigkeit während des Rückführens das Erhitzen der Flüssigkeit entlang eines Abschnitts des Vorwasch-Zuführzweigs (35) umfasst.

4. System (10) zum Waschen von Behältern für pharmazeutische Zwecke, umfassend:
mindestens eine Waschstation (13), umfassend eine Vielzahl von Waschdüsen (16) und eine Sammelvorrichtung (17) zum Sammeln der aus den Waschdüsen (16) abgegebenen Flüssigkeit;
mindestens eine Vorwaschstation (12), umfassend eine Vielzahl von Vorwaschdüsen (14) und eine Sammelvorrichtung (15) zum Sammeln von aus den Vorwaschdüsen (14) abgegebener Flüssigkeit;
einen Flüssigkeitsrückgewinnungstank (19);
einen Waschkreislauf (21), der eine Flüssigkeitsquelle (18) in Fluidverbindung mit den Waschdüsen (16) bringt und die Flüssigkeitssammelvorrichtung (17) der mindestens einen Waschstation (13) in Fluidverbindung mit dem Rückgewinnungstank (19) bringt;
einen Vorwaschkreislauf (34), der einen Vorwasch-Zuführzweig (35), der in Fluidverbindung mit dem Rückgewinnungstank (19) steht und wahlweise in Fluidverbindung mit den Vorwaschdüsen (14) steht, sowie einen Vorwasch-Rücklaufzweig (48) umfasst, der die Flüssigkeitssammelvorrichtung (15) der mindestens einen Vorwaschstation (12) in Fluidverbindung mit einem Abfluss (20) bringt;
einen Flüssigkeitserhitzer (41), der an einem Abschnitt des Vorwasch-Zuführzweigs (35) angeordnet und so ausgelegt ist, dass er die in diesem Abschnitt des Vorwasch-Zuführzweigs (35) strömende Flüssigkeit erhitzt;
**dadurch gekennzeichnet, dass**
der Vorwaschkreislauf (34) ferner einen Rückführzweig (38) umfasst, die in Fluidverbindung mit dem Rückgewinnungstank (19) steht und wahlweise in Fluidverbindung mit der Vorwasch-Zuführzweig (35) steht;
und dadurch, dass
wenn der Rückführzweig (38) in Fluidverbindung mit dem Vorwasch-Zuführzweig (35) steht, der Vorwasch-Zuführzweig (35) nicht in Fluidverbindung mit den Vorwaschdüsen (14) steht.

5. Waschsystem (10) nach Anspruch 4, wobei der Waschkreislauf (21) einen Waschzuführzweig (22) umfasst, der wahlweise in Fluidverbindung mit der Flüssigkeitsquelle (18) steht und in Fluidverbindung mit den Waschdüsen (16) steht, sowie einen Waschrücklaufzweig (33), der die Flüssigkeitssammelvorrichtung (17) der mindestens einen Waschstation (13) in Fluidverbindung mit dem Rückgewinnungstank (19) bringt.

6. Waschsystem (10) nach Anspruch 5, wobei, wenn der Rückführzweig (38) in Fluidverbindung mit dem Vorwasch-Zuführzweig (35) steht, der Waschzuführzweig (22) nicht in Fluidverbindung mit der Flüssigkeitsquelle (18) steht.

7. Waschsystem (10) nach einem der Ansprüche 4 bis 6, wobei die Flüssigkeitsquelle (18) so ausgelegt ist, dass sie auf eine vorbestimmte Temperatur erhitzte Flüssigkeit abgibt.

8. Waschsystem (10) nach einem der Ansprüche 4 bis 7, wobei zwischen dem Rückführzweig (38) und dem Vorwasch-Zuführzweig (35) ein Rückführventil (45) vorgesehen ist, das zwischen einer offenen Stellung, in der es den Vorwasch-Zuführzweig (35) in Fluidverbindung mit dem Rückführzweig (38) bringt, und einer geschlossenen Stellung umschaltbar ist, in der es die Fluidverbindung zwischen dem Vorwasch-Zuführzweig (35) und dem Rückführzweig (38) unterbricht.

9. Waschsystem (10) gemäß einem der Ansprüche 4 bis 8, wobei der Vorwasch-Zuführzweig (35) eine Vorwasch-Hauptleitung (36), die in Fluidverbindung mit dem Rückgewinnungstank (19) steht, und mindestens eine Vorwasch-Abgabeleitung (37) umfasst, die in Fluidverbindung mit der Vorwasch-Hauptleitung (36) und mit den Vorwaschdüsen (14) steht; wobei ein Vorwasch-Abgabeventil (44) an der Vorwasch-Abgabeleitung (37) angeordnet ist und zwischen einer offenen Stellung, in der es die Vorwasch-Hauptleitung (36) in Fluidverbindung mit der Vorwasch-Abgabeleitung (37) bringt, und einer geschlossenen Stellung, in der es die Fluidverbindung zwischen der Vorwasch-Hauptleitung (36) und der Vorwasch-Abgabeleitung (37) unterbricht, umschaltbar ist.

10. Waschsystem (10) nach den Ansprüchen 8 und 9, wobei sich das Vorwasch-Abgabeventil (44) in der geschlossenen Stellung befindet, wenn sich das Rückführventil (45) in der offenen Stellung befindet, und sich das Vorwasch-Abgabeventil (44) in der offenen Stellung befindet, wenn sich das Rückführventil (45) in der geschlossenen Stellung befindet.

11. Waschsystem (10) nach Anspruch 4 und 9, wobei der Flüssigkeitserhitzer (41) an der Vorwasch-Hauptleitung (36) des Vorwasch-Zuführzweigs (35) angeordnet ist.

12. Waschsystem (10) gemäß einem der Ansprüche 4 bis 11, wobei der Waschkreislauf (21) und der Vorwaschkreislauf (34) ausschließlich über den Rückgewinnungstank (19) in Fluidverbindung miteinander stehen.

## Revendications

1. Procédé de lavage de récipients (100) à usage pharmaceutique, comprenant :
l'acheminement d'un fluide chauffé vers des buses de lavage (16) d'au moins une station de lavage (13) par l'intermédiaire d'un circuit de lavage (21) ;
l'acheminement, vers un réservoir de récupération (19), du fluide évacué par lesdites buses de lavage (16) par l'intermédiaire dudit circuit de lavage (21) ;
l'acheminement du fluide provenant dudit réservoir de récupération (19) vers des buses de prélavage (14) d'au moins une station de prélavage (12) par l'intermédiaire d'une branche de distribution pour le prélavage (35) d'un circuit de prélavage (34) ;
dans lequel, en cas d'arrêt temporaire du système de lavage, il est prévu de :
interrompre l'acheminement de fluide vers les buses de prélavage (14) ; **caractérisé par**
la mise en communication fluidique d'une branche de recirculation (38) du circuit de prélavage (34) avec ladite branche de distribution pour le prélavage (35) et avec ledit réservoir de récupération (19) ;
la recirculation du fluide entre le réservoir de récupération (19), la branche de distribution pour le prélavage (35) et la branche de recirculation (38), et le chauffage dudit fluide pendant sa recirculation.

2. Procédé selon la revendication 1, dans lequel, en cas d'arrêt temporaire du système de lavage, il est prévu d'interrompre l'acheminement de fluide vers les buses de lavage (16).

3. Procédé selon la revendication 1 ou 2, dans lequel le chauffage dudit fluide pendant sa recirculation comprend le chauffage dudit fluide le long d'une partie de ladite branche de distribution pour le prélavage (35).

4. Système (10) de lavage de récipients à usage pharmaceutique comprenant :
au moins une station de lavage (13) comprenant une pluralité de buses de lavage (16) et un dispositif de collecte (17) destiné à collecter le fluide distribué par lesdites buses de lavage (16) ;
au moins une station de prélavage (12) comprenant une pluralité de buses de prélavage (14) et un dispositif de collecte (15) destiné à collecter le fluide distribué par lesdites buses de prélavage (14) ;
un réservoir de récupération de fluide (19) ;
un circuit de lavage (21) qui met une source de fluide (18) en communication fluidique avec lesdites buses de lavage (16) et qui met ledit dispositif de collecte de fluide (17) de ladite au moins une station de lavage (13) en communication fluidique avec ledit réservoir de récupération (19) ;
un circuit de prélavage (34) comprenant une branche de distribution pour le prélavage (35) mise en communication fluidique avec ledit réservoir de récupération (19) et mise sélectivement en communication fluidique avec lesdites buses de prélavage (14), ainsi qu'une branche de retour pour le prélavage (48) mettant ledit dispositif de collecte de fluide (15) de ladite au moins une station de prélavage (12) en communication fluidique avec un drain (20) ;
un réchauffeur de fluide (41) mis sur une partie de ladite branche de distribution pour le prélavage (35) et configuré pour chauffer le fluide s'écoulant dans ladite partie de la branche de distribution pour le prélavage (35) ;
**caractérisé en ce que**
ledit circuit de prélavage (34) comprend en outre une branche de recirculation (38) mise en communication fluidique avec ledit réservoir de récupération (19) et mise de manière sélective en communication fluidique avec ladite branche de distribution pour le prélavage (35) ;
et **en ce que**
lorsque ladite branche de recirculation (38) est mise en communication fluidique avec ladite branche de distribution pour le prélavage (35), ladite branche de distribution pour le prélavage (35) n'est pas mise en communication fluidique avec lesdites buses de prélavage (14).

5. Système de lavage (10) selon la revendication 4, dans lequel ledit circuit de lavage (21) comprend une branche de distribution pour le lavage (22) mise de manière sélective en communication fluidique avec ladite source de fluide (18) et mise en communication fluidique avec lesdites buses de lavage (16) et une branche de retour de lavage (33) qui met ledit dispositif de collecte de fluide (17) de ladite au moins une station de lavage (13) en communication fluidique avec ledit réservoir de récupération (19).

6. Système de lavage (10) selon la revendication 5, dans lequel, lorsque ladite branche de recirculation (38) est mise en communication fluidique avec ladite branche de distribution pour le prélavage (35), ladite branche de distribution pour le lavage (22) n'est pas mise en communication fluidique avec ladite source de fluide (18).

7. Système de lavage (10) selon l'une quelconque des revendications 4 à 6, dans lequel ladite source de fluide (18) est configurée pour distribuer un fluide chauffé à une température prédéterminée.

8. Système de lavage (10) selon l'une quelconque des revendications 4 à 7, dans lequel, entre ladite branche de recirculation (38) et ladite branche de distribution pour le prélavage (35) est prévue une soupape de recirculation (45) pouvant basculer entre une position ouverte, dans laquelle elle met ladite branche de distribution pour le prélavage (35) en communication fluidique avec ladite branche de recirculation (38), et une position fermée, dans laquelle elle interrompt la communication fluidique entre ladite branche de distribution pour le prélavage (35) et ladite branche de recirculation (38).

9. Système de lavage (10) selon l'une quelconque des revendications 4 à 8, dans lequel ladite branche de distribution pour le prélavage (35) comprend une conduite principale de prélavage (36) reliée fluidiquement audit réservoir de récupération (19) et au moins une conduite de distribution pour le prélavage (37) reliée fluidiquement à ladite conduite principale pour le prélavage (36) et auxdites buses de prélavage (14) ; une soupape de distribution de prélavage (44) étant mise sur ladite conduite de distribution pour le prélavage (37) et pouvant être commutée entre une position ouverte dans laquelle elle met ladite conduite principale pour le prélavage (36) en communication fluidique avec ladite conduite de distribution pour le prélavage (37) et une position fermée dans laquelle elle interrompt la communication fluidique entre ladite conduite principale pour le prélavage (36) et ladite conduite de distribution pour le prélavage (37).

10. Système de lavage (10) selon les revendications 8 et 9, dans lequel, lorsque ladite soupape de recirculation (45) est en position ouverte, ladite soupape de distribution pour le prélavage (44) est en position fermée, et lorsque ladite soupape de recirculation (45) est en position fermée, ladite soupape de distribution pour le prélavage (44) est en position ouverte.

11. Système de lavage (10) selon les revendications 4 et 9, dans lequel ledit réchauffeur de fluide (41) est mis sur ladite conduite principale pour le prélavage (36) de ladite branche de distribution pour le prélavage (35).

12. Système de lavage (10) selon l'une quelconque des revendications 4 à 11, dans lequel ledit circuit de lavage (21) et ledit circuit de prélavage (34) sont en communication fluidique l'un avec l'autre uniquement par l'intermédiaire dudit réservoir de récupération (19).
